# EUROPEAN PATENT APPLICATION

(11) **EP 3 821 889 A1**
(43) Date of publication of application: **19.05.2021**
(21) Application number: 19209545.3
(22) Date of filing: 15.11.2019
(51) Int. Cl.: A61K 31/165, A61P 9/10

(54) **METHODS FOR USING LOW-DOSE COLCHICINE AFTER MYOCARIAL INFARCTION**

(71) Applicant: Institut de Cardiologie de Montréal, Montréal, QC H1T 1C8 (CA)
(72) Inventor: Tardif, Jean-Claude, Montreal, Québec H1T 1C8 (CA)
(74) Representative: Weickmann & Weickmann PartmbB

(57) **Abstract**

The invention relates to colchicine for use in a method of treating a patient after having a myocardial infarction (MI), the method including initiating the administration of colchicine at a daily low dose to the patient within about 30 days of the MI.

## Description

### Background of the Invention

The invention relates to a treatment regimen for patients after suffering a myocardial infarction.

Inflammation appears to play an important role in atherosclerosis (Hansson GK, Inflammation, atherosclerosis, and coronary artery disease. N Engl J Med 2005;352:1685-95). Inhibition of interleukin-1β by the injectable monoclonal antibody canakinumab led to a 15% lower risk of cardiovascular events than was observed with placebo in the Canakinumab Antiinflammatory Thrombosis Outcomes Study (CANTOS) but also led to a slightly higher incidence of fatal infections (Ridker PM et al., Antiinflammatory therapy with canakinumab for atherosclerotic disease. N Engl J Med 2017;377:1119-31). In contrast, methotrexate did not affect cardiovascular outcomes or plasma markers of inflammation in the Cardiovascular Inflammation Reduction Trial (CIRT) (Ridker PM et al., Low-dose methotrexate for the prevention of atherosclerotic events. N Engl J Med 2019;380:752-62). Considering these differing results and given that canakinumab is not clinically available for cardiovascular prevention, the search for a widely used alternative antiinflammatory treatment that may reduce the risk of atherosclerotic events among patients with coronary artery disease continues.

Colchicine is an inexpensive, orally administered, potent anti-inflammatory medication that was initially extracted from the autumn crocus and has been used for centuries. Its mechanism of action is through the inhibition of tubulin polymerization and microtubule generation and, possibly, effects on cellular adhesion molecules, inflammatory chemokines, and the inflammasome (Ravelli RB et al., Insight into tubulin regulation from a complex with colchicine and a stathmin-like domain. Nature 2004;428:198-202; Perico N et al., Colchicine interferes with L-selectin and leukocyte function-associated antigen-1 expression on human T lymphocytes and inhibits T cell activation. J Am Soc Nephrol 1996; 7:594-601; Pope RM and Tschopp J, The role of interleukin-1 and the inflammasome in gout: implications for therapy. Arthritis Rheum 2007; 56:3183-8). Colchicine is currently indicated for the treatment of gout, familial Mediterranean fever, and pericarditis (Cerquaglia C et al., Pharmacological and clinical basis of treatment of Familial Mediterranean Fever (FMF) with colchicine or analogues: an update. Curr Drug Targets Inflamm Allergy 2005;4:117-24; and Imazio M et al., Colchicine in addition to conventional therapy for acute pericarditis: results of the COlchicine for acute PEricarditis (COPE) trial. Circulation 2005;112:2012-6).

In the Low-Dose Colchicine (LoDoCo) trial, patients with stable coronary disease treated with colchicine at a dose of 0.5 mg once daily had fewer cardiovascular events than those not receiving colchicine (Nidorf SM et al., Low-dose colchicine for secondary prevention of cardiovascular disease. J Am Coll Cardiol 2013;61:404-10). However, that trial enrolled only 532 patients and was not placebo-controlled.

Because acute coronary syndromes are associated with higher risks of recurrent events and exacerbated inflammation a need exists in the art for new treatment regimens.

### Summary of the Invention

As is disclosed herein, a clinical trial referred to as the Colchicine Cardiovascular Outcomes Trial (COLCOT) was conducted to evaluate the effects of colchicine on cardiovascular outcomes as well as its long-term safety profile in patients who had recently had a myocardial infarction.

Our results evidence that the use of colchicine in patients who have recently had a myocardial infarction significantly improved their quality of life in several ways. Colchicine, for example, at a daily low dose of 0.5 mg led to a statistically significant lower risk of ischemic cardiovascular events than placebo. Death from cardiovascular causes, resuscitated cardiac arrest, myocardial infarction, stroke, or urgent hospitalization for angina leading to coronary revascularization was also significantly lower among the patients who received 0.5 mg of colchicine than those who received placebo. Indeed, the methods described herein surprisingly and significantly reduce patient morbidity. For example, patients receiving low dose colchicine had reduced morbidity relative to placebo, as is demonstrated by the rates of the respective primary composite end points for the two patient populations (P = 0.02). This reduction in morbidity was particularly prominent among colchicine-receiving patients in the reduction of severe conditions, such as stroke and urgent hospitalization for angina leading to revascularization: hazard ratios are 0.26 (95% confidence interval is 0.10-0.70) and 0.50 (95% confidence interval is 0.31-0.81), respectively. Still further, unlike the use of the anti-inflammatory canakinumab for atherosclerotic events, colchicine did not increase the incidence of septic shock. Moreover, no serious adverse event of myopathy linked to colchicine occurred despite the use of statins in 99% of trial participants.

In view of the aforementioned, the invention, in one aspect, relates to colchicine for use in a method of treating a patient after having a myocardial infarction, the method including initiating the administration of colchicine at a daily low dose to the patient within about 30 days of the myocardial infarction.

In some embodiments, the method involves administering colchicine within 5, 10, 15, 20, or 25 days of the MI. In some embodiments, the method includes administering colchicine within 10 days of the myocardial infarction. In still other embodiments, the method includes administering colchicine within 15 days of the myocardial infarction. In yet other embodiments, the method includes administering colchicine at about 30 days of the myocardial infarction (for example, at 31, 32, or 33 days).

In some embodiments, the patient administered colchicine received percutaneous coronary intervention for treating the patient's myocardial infarction.

In some embodiments, the patient administered was previously prescribed a medication (for example, an antiplatelet agent, a statin, aspirin or a combination thereof).

In some embodiments, the administration of colchicine is initiated upon assessment in (a) an emergency department (ED), (b) the hospital, or (c) a medical office setting.

In some embodiments, colchicine is administered in the form of a tablet or a capsule.

In some embodiments, colchicine is administered at 0.3 to 0.7 mg. For example, colchicine is administered at 0.4 to 0.6 mg, and is preferably administered at 0.5 mg. In some embodiments, colchicine is administered in a 0.5 mg tablet or a 0.6 mg tablet. In other embodiments, colchicine is administered in a 0.25 mg tablet.

In some embodiments, colchicine is administrated once, twice, or three times daily.

Various colchicine formulations are readily available and well known in the art.

Colchicine for use in a method of treating a patient after having a myocardial infarction typically continues, as needed, throughout the life of a patient. For example, in some embodiments, the duration of treatment is for 6 months, 12 months, 18 months, 24 months, 30 months, 36 months, or even longer as is needed.

Other features and advantages of the invention will be apparent from the following Detailed Description, the Drawings, and the Claims.

### Brief Description of the Drawings

FIG. 1 shows a flow chart of the randomization and follow-up of patients in the study.
FIG. 2 shows the cumulative incidence of cardiovascular events (intention-to-treat population). Shown are the Kaplan-Meier event curves for the primary efficacy composite end point of death from cardiovascular causes, resuscitated cardiac arrest, myocardial infarction, stroke, or urgent hospitalization for angina leading to coronary revascularization in the colchicine group and the placebo group in a time-to-event analysis. The inset shows the same data on an enlarged y axis.

### Detailed Description of the Invention

We performed a randomized, double-blind trial involving patients recruited within 30 days after a myocardial infarction. The patients were randomly assigned to receive either low-dose colchicine (0.5 mg once daily) or placebo. A total of 4745 patients were enrolled; 2366 patients were assigned to the colchicine group, and 2379 to the placebo group. Patients were followed for a median of 22.6 months.

The primary efficacy end point was a composite of death from cardiovascular causes, resuscitated cardiac arrest, myocardial infarction, stroke, or urgent hospitalization for angina leading to coronary revascularization. The components of the primary end point and safety were also assessed.

The primary end point occurred in 5.5% of the patients in the colchicine group, as compared with 7.1% of those in the placebo group (hazard ratio, 0.77; 95% confidence interval [CI], 0.61 to 0.96; P = 0.02). The hazard ratios were 0.84 (95% Cl, 0.46 to 1.52) for death from cardiovascular causes, 0.83 (95% Cl, 0.25 to 2.73) for resuscitated cardiac arrest, 0.91 (95% Cl, 0.68 to 1.21) for myocardial infarction, 0.26 (95% Cl, 0.10 to 0.70) for stroke, and 0.50 (95% Cl, 0.31 to 0.81) for urgent hospitalization for angina leading to coronary revascularization. Diarrhea was reported in 9.7% of the patients in the colchicine group and in 8.9% of those in the placebo group (P = 0.35). Pneumonia was reported as a serious adverse event in 0.9% of the patients in the colchicine group and in 0.4% of those in the placebo group (P = 0.03).

### METHODS

### Trial Design and Oversight

In this randomized, double-blind, placebo-controlled, investigator-initiated trial, we assigned patients in a 1:1 ratio to receive either colchicine (at a dose of 0.5 mg once daily) or placebo. The trial protocol, available at NEJM.org, was designed by a trial steering committee. The protocol was approved by the institutional review board at each of the 167 centers in the 12 countries that participated in the trial (available at NEJM.org). All trial support activities, including project coordination, data management, site monitoring, and statistical oversight and analyses, were performed at the Montreal Health Innovations Coordinating Center. Potential trial end-point events were adjudicated by an independent clinical end-point committee composed of experienced cardiologists and neurologists who were unaware of the trial-group assignments. The trial was overseen by a data and safety monitoring board of independent experts. The trial medication and matching placebo were provided by Pharmascience.

### Trial Population

Adult patients were eligible if they had had a myocardial infarction within 30 days before enrollment, had completed any planned percutaneous revascularization procedures, and were treated according to national guidelines that included the intensive use of statins.

Patients were excluded if they had severe heart failure, a left ventricular ejection fraction of less than 35%, stroke within the previous 3 months, a type 2 index myocardial infarction, coronary-bypass surgery either within the previous 3 years or planned, a history of noncutaneous cancer within the previous 3 years, inflammatory bowel disease or chronic diarrhea, neuromuscular disease or a nontransient creatine kinase level that was greater than three times the upper limit of the normal range (unless due to infarction), clinically significant nontransient hematologic abnormalities, severe renal disease with a serum creatinine level that was greater than two times the upper limit of the normal range; severe hepatic disease, drug or alcohol abuse, current or planned long-term systemic glucocorticoid therapy, or a history of clinically significant sensitivity to colchicine. (Details regarding eligibility criteria are provided herein.)

Written informed consent was obtained from all the patients before enrollment. Clinical evaluations occurred at 1 month and 3 months after randomization and every 3 months thereafter.

### End Points

The primary efficacy end point was a composite of death from cardiovascular causes, resuscitated cardiac arrest, myocardial infarction, stroke, or urgent hospitalization for angina leading to coronary revascularization in a time-to-event analysis.

The secondary end points consisted of the components of the primary efficacy end point; a composite of death from cardiovascular causes, resuscitated cardiac arrest, myocardial infarction, or stroke; and total mortality in time-to-event analyses. Coronary revascularization, hospitalization for heart failure, atrial fibrillation, and deep venous thrombosis or pulmonary embolus were prespecified as exploratory end points in the protocol.

Additional prespecified exploratory end points included the change from baseline to 6 months in the high-sensitivity C-reactive protein level and the change from baseline to 12 months in the white-cell count. The C-reactive protein biomarker substudy was implemented after a protocol amendment and was optional for sites and for patients; 34 sites chose to participate in this substudy.

All serious adverse events were recorded. The only other adverse events recorded were those that were considered to be related to the gastrointestinal system, events that were judged by the investigator to be related to colchicine or placebo, or laboratory abnormalities that had been judged by the investigator to be clinically significant.

### Statistical Analysis

In this event-driven trial, it was estimated that a sample of approximately 4500 patients undergoing randomization (with 2250 patients in each group) or, in terms of events, a total number of 301 patients with a first positively adjudicated primary end-point event would yield adequate power. The sample-size calculation was based on the primary efficacy end point and assumed a 27% lower risk with colchicine than with placebo, indicated by a hazard ratio of 0.724. With the use of a two-sided test at the 0.05 significance level, the trial would have 80% power if it continued until 301 positively adjudicated primary events occurred in the combined trial groups. The trial design assumed an event rate of 7% in the placebo group at 24 months, an 18-month recruitment period during which patients would be uniformly recruited, a 24-month minimum follow-up period, and a 1% annual rate of loss to follow-up or withdrawal of consent.

The efficacy analyses were conducted with the use of positively adjudicated data and according to the intention-to-treat principle. The primary end point was compared between the two trial groups with the use of a log-rank test, and the hazard ratio from a Cox proportional-hazards model, with a 95% confidence interval, was calculated. A Cox proportional-hazards model with adjustment for important baseline characteristics was also used as prespecified in the protocol.

The analysis of the primary end point was repeated in the per-protocol population (i.e., patients without major protocol deviations). Secondary and exploratory end points expressed as time to event were analyzed similarly. The changes from baseline to follow-up were analyzed with the use of an analysis of covariance model with adjustment for baseline value, and estimates of treatment effect are presented with 95% confidence intervals.

The efficacy end points expressed as time to event could be assessed in all patients because the event dates and censoring dates were complete, with the exception of one incomplete event date for atrial fibrillation; therefore, imputation for missing data was not done.

In the analysis of time to event, the following censoring rules were used. For death from any cause and death from cardiovascular causes, data from event-free patients who completed the trial were censored at the date of trial completion, and data from patients who did not complete the trial, such as those who were lost to follow-up or who withdrew consent, were censored at the date of last contact or the date of the assessment of survival status, whichever was later.

For the analysis of death from cardiovascular causes, patients who died from a noncardiovascular cause had their data censored at the time of death.

For all other end points, including the primary end point, the same censoring rules applied, but the survival status was not used because no formal assessment of end points was done at the assessment of survival status.

An analysis of the components of the primary end point with death from noncardiovascular causes as a competing event for death from cardiovascular causes, and with death from any cause as a competing event for the other components, was conducted with the use of the Fine and Gray subdistribution hazard model (Fine JP and Gray RJ, A proportional hazards model for the subdistribution of a competing risk. J Am Stat Assoc 1999;94:496-509). No missing data were imputed except for age (see notes to Tables *infra*).

To account for the occurrence of multiple primary end-point events within patients, recurrent-event analyses were undertaken with the use of negative binomial regression, Andersen-Gill, and Wei-Lin-Weissfeld models (Rogers JK et al., Analysing recurrent hospitalizations in heart failure: a review of statistical methodology, with application to CHARM-Preserved. Eur J Heart Fail 2014;16:33-40; Andersen PK and Gill RD, Cox's regression model for counting processes: a large sample study. Ann Stat 1982;10:1100-20; Lin DY and Wei LJ, The robust inference for the proportional hazards model. J Am Stat Assoc 1989;84:1074-8; Lin DY et al. Semiparametric regression for the mean and rate functions of recurrent events. J R Stat Soc 2000;62:711-30; Wei LJ and Glidden DV, An overview of statistical methods for multiple failure time data in clinical trials. Stat Med 1997;16:833-9; Ghosh D, Methods for analysis of multiple events in the presence of death. Control Clin Trials 2000;21:115-26; Li QH and Lagakos SW, Use of the Wei-Lin-Weissfeld method for the analysis of a recurring and a terminating event. Stat Med 1997;16:925-40; Metcalfe C and Thompson SG, The importance of varying the event generation process in simulation studies of statistical methods for recurrent events. Stat Med 2006;25:165-79; Jahn-Eimermacher A, Comparison of the Andersen-Gill model with Poisson and negative binomial regression on recurrent event data. Comput Stat Data Anal 2008;52:4989-97).

An interim analysis was performed after 50% of the primary end-point events had been positively adjudicated. The prespecified stopping rule for efficacy was based on the Lan-DeMets procedure with the O'Brien-Fleming alpha-spending function. After review of the interim results, the data and safety monitoring board recommended that the trial should continue as planned.

To account for this interim analysis, the statistical significance level was set to 0.0490 for the final analysis of the primary end point. All other statistical tests were two-sided and conducted at the 0.05 significance level. Statistical analyses were performed with the use of SAS software, version 9.4 (SAS Institute). There was no prespecified plan to adjust for multiple comparisons across the multiple methods that were used to analyze the primary and secondary end points; results of these analyses are reported with point estimates and 95% confidence intervals, without P values. The 95% confidence intervals were not adjusted for multiple comparisons, and inferences drawn from them may not be reproducible.

The final amendment to the statistical analysis plan was approved on August 28, 2019, before unblinding of the trial-group assignments occurred.

### RESULTS

### Patients

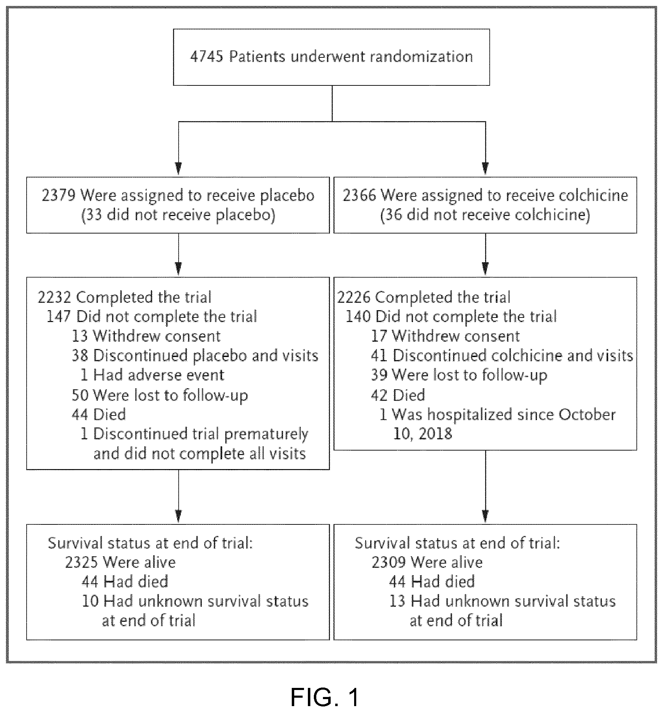

Trial enrollment began in December 2015 and was completed in August 2018; the last trial visit was in July 2019. A total of 4745 patients underwent randomization (with 2366 being assigned to the colchicine group and 2379 to the placebo group) and were followed for a median of 22.6 months. At the time of the database lock on August 28, 2019, and unblinding on August 29, 2019, vital status was available for all except 23 patients (99.5%); 89 patients (1.9%) were lost to follow-up, and 30 patients (0.6%) withdrew consent. Details regarding the disposition of the patients are provided in Figure 1.

The characteristics of the patients at baseline are shown in Table 1.

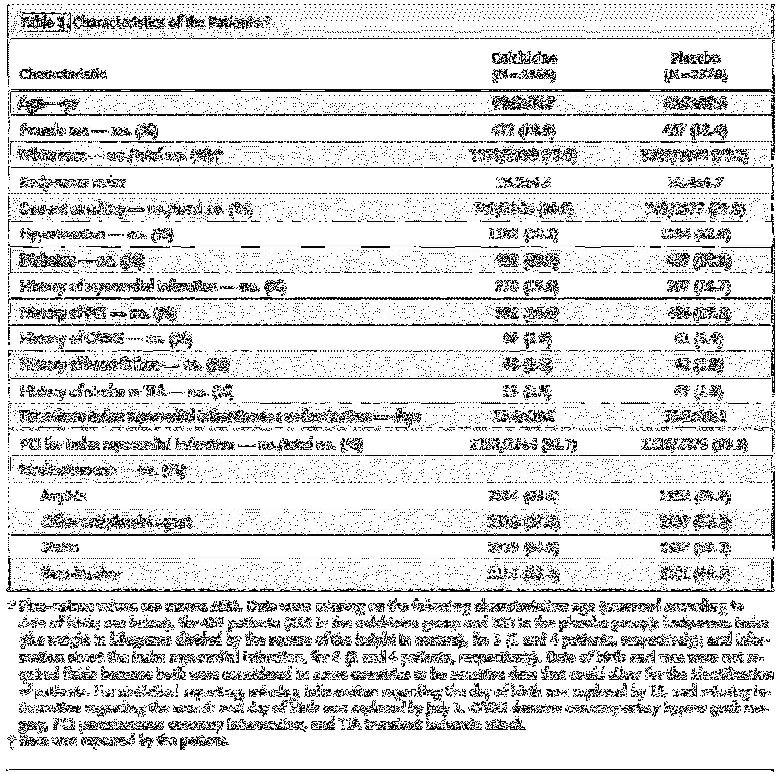

Patients were enrolled a mean of 13.5 days after myocardial infarction. The mean age of the patients was 60.6 years, 19.2% of the patients were women, and 20.2% had diabetes. Most patients (93.0%) underwent percutaneous coronary intervention for their index myocardial infarction. Aspirin, a different antiplatelet agent, and a statin were taken by 98.8%, 97.9% and 99.0% of the patients, respectively.

At the end of the trial, the trial regimen had been discontinued in 18.4% of the patients in the colchicine group and in 18.7% of those in the placebo group. Among the patients who discontinued the trial regimen, the median time of taking the trial drug was 7.1 months (interquartile range, 1.9 to 14.6) in the colchicine group, as compared with 6.1 months (interquartile range, 1.6 to 14.4) in the placebo group. Overall, the median duration of receipt of the trial drug was 19.6 months in the colchicine group and 19.5 months in the placebo group.

### Clinical Efficacy End Points

A primary end-point event occurred in 5.5% of the patients in the colchicine group, as compared with 7.1% of those in the placebo group (hazard ratio, 0.77; 95% confidence interval [CI], 0.61 to 0.96; P = 0.02 by the log-rank test). A multivariable Cox regression model with adjustment for baseline covariates yielded a similar result (Table 2).

**Table 2. Multivariable Cox Regression Model for Time to First Primary Endpoint.**

| **Effect** | | **Adjusted Hazard Ratio (95% CI)** | **P Value** |
|---|---|---|---|
| Randomized treatment group | Colchicine vs. Placebo | 0.78 (0.62-0.98) | 0.03 |
| Age at randomization (years) | | 1.02 (1.01-1.03) | <0.001 |
| History of diabetes | Yes vs. No | 1.86 (1.46-2.37) | <0.001 |
| Prior coronary revascularization (PCI or CABG) | Yes vs. No | 2.02 (1.58-2.58) | <0.001 |
| Prior heart failure | Yes vs. No | 1.81 (1.08-3.04) | 0.03 |

| | | | |
|---|---|---|---|
| CABG denotes coronary artery bypass graft, and PCI percutaneous coronary intervention. The model was based on 4745 observations. All baseline characteristics that showed an association (P<0.20) with the occurrence of a first positively adjudicated primary endpoint were included in the stepwise multivariable Cox regression. For age at randomization, the hazard ratio is for an increase of one year of age. | | | |

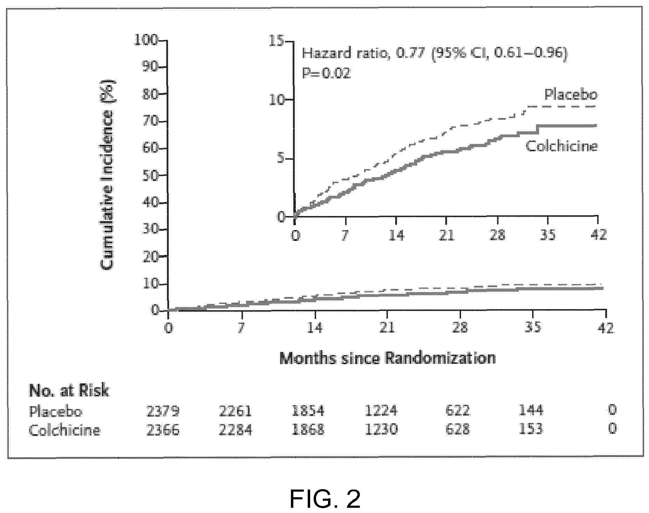

The event curves that were based on a Kaplan-Meier analysis of the primary efficacy end point are shown in Figure 2.

In the prespecified per-protocol analysis involving patients who adhered to the protocol, the primary end point occurred in 5.1% of the patients in the colchicine group and in 7.1% of those in the placebo group (hazard ratio, 0.71; 95% Cl, 0.56 to 0.90) (Table 3).

**Table 3. Rates and Hazard Ratios for the Primary Endpoint and its Components in the Per-Protocol Population†.**

| **Clinical Outcome** | **Colchicine** | **Placebo** | **Hazard Ratio (95% CI)** |
|---|---|---|---|
| Per-protocol population | N=2260 | N=2270 | |
| | | | |
| Primary endpoint - no. (%) | 115(5.1%) | 162(7.1%) | 0.71 (0.56-0.90) |
| CV death - no. (%) | 19 (0.8%) | 23 (1.0%) | 0.83 (0.45-1.53) |
| Resuscitated cardiac arrest - no. (%) | 5 (0.2%) | 5 (0.2%) | 1.00 (0.29-3.46) |
| MI- no. (%) | 77 (3.4%) | 92(4.1%) | 0.84 (0.62-1.14) |
| Stroke - no. (%) | 5(0.2%) | 19(0.8%) | 0.26 (0.10-0.71) |
| Urgent hospitalization for angina requiring revascularization - no. (%) | 22 (1.0%) | 47 (2.1%) | 0.47 (0.28-0.78) |

| | | | |
|---|---|---|---|
| CV denotes cardiovascular, and MI myocardial infarction. †The per-protocol population consisted of patients without major protocol deviations. | | | |

Table 4 shows the percentages of patients with events and the hazard ratios for the components of the primary end point, including death from cardiovascular causes (hazard ratio, 0.84; 95% Cl, 0.46 to 1.52), resuscitated cardiac arrest (hazard ratio, 0.83; 95% Cl, 0.25 to 2.73), myocardial infarction (hazard ratio, 0.91; 95% Cl, 0.68 to 1.21), stroke (hazard ratio, 0.26; 95% Cl, 0.10 to 0.70), and urgent hospitalization for angina leading to coronary revascularization (hazard ratio, 0.50; 95% Cl, 0.31 to 0.81). The hazard ratios remained unchanged in the analysis that took competing events into account.

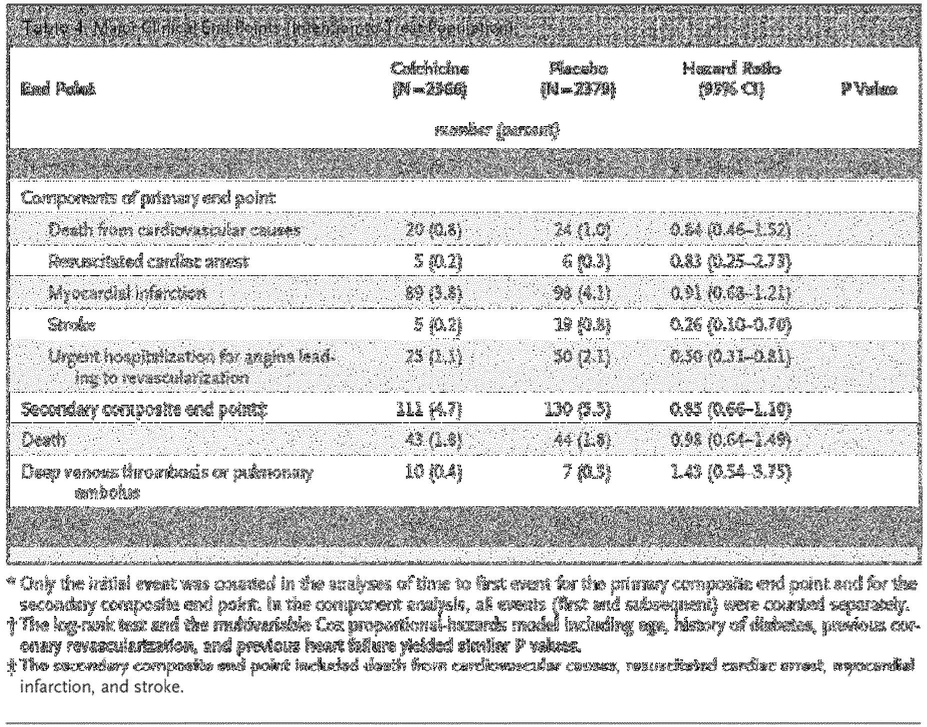

The secondary efficacy end point consisting of a composite of death from cardiovascular causes, cardiac arrest, myocardial infarction, or stroke occurred in 4.7% of the patients in the colchicine group and in 5.5% of those in the placebo group (hazard ratio, 0.85; 95% Cl, 0.66 to 1.10). Data on the primary, secondary, and exploratory efficacy end points are provided in Table 4. Two patients had a first positively adjudicated event of urgent hospitalization for angina leading to coronary revascularization within 14 days after randomization. The median time to this clinical end point was 258 days.

Efficacy results in prespecified subgroups are shown in Table 5. The total number of primary end-point events (first and recurrent) was 154 in the colchicine group and 223 in the placebo group, over periods of 52,949 and 53,060 patient-months of follow-up, respectively. Thus, the primary end-point event rates per 100 patient-months were 0.29 in the colchicine group and 0.42 in the placebo group (rate ratio, 0.66; 95% Cl, 0.51 to 0.86) (Table 6).

**Table 5. Primary Efficacy Composite Endpoint in Prespecified Subgroups†.**

| **Subgroup** | **Colchicine** | **Placebo** | **Hazard ratio (95% CI)** |
|---|---|---|---|
| *no. of patients with event*/*total no. of patients (%)* | | | |
| All patients | 131/2366 (5.5%) | 170/2379 (7.1%) | 0.77 (0.61-0.96) |
| | | | |
| Smoking | | | |
| Non-smoker | 47/787 (6.0%) | 52/797 (6.5%) | 0.90 (0.61; 1.34) |
| Previous smoker | 46/871 (5.3%) | 77/872 (8.8%) | 0.59 (0.41; 0.85) |
| Active smoker | 38/708 (5.4%) | 41/708 (5.8%) | 0.93 (0.60; 1.44) |
| History of diabetes | | | |
| Yes | 40/462 (8.7%) | 65/497 (13.1%) | 0.65 (0.44; 0.96) |
| No | 91/1904 (4.8%) | 105/1882 (5.6%) | 0.85 (0.64; 1.13) |
| History of hypertension | | | |
| Yes | 83/1185 (7.0%) | 112/1236 (9.1%) | 0.76 (0.57; 1.01) |
| No | 48/1181 (4.1%) | 58/1143 (5.1%) | 0.80 (0.54; 1.17) |
| Prior MI | | | |
| Yes | 46/370 (12.4%) | 47/397 (11.8%) | 1.05 (0.70; 1.58) |
| No | 85/1996 (4.3%) | 123/1982 (6.2%) | 0.68 (0.51; 0.89) |
| Prior PCI or CABG | | | |
| Yes | 48/419 (11.5%) | 57/447 (12.8%) | 0.91 (0.62; 1.34) |
| No | 83/1947 (4.3%) | 113/1932 (5.8%) | 0.72 (0.54; 0.95) |
| Prior stroke or TIA | | | |
| Yes | 8/55 (14.5%) | 9/67 (13.4%) | 1.09 (0.42; 2.82) |
| No | 123/2311 (5.3%) | 161/2312 (7.0%) | 0.76 (0.60; 0.96) |
| Sex‡ | | | |
| Male | 94/1894 (5.0%) | 135/1942 (7.0%) | 0.70 (0.54; 0.91) |
| Female | 37/472 (7.8%) | 35/437 (8.0%) | 0.99 (0.63; 1.58) |
| White blood cell count¶ | | | |
| Below median | 41/660 (6.2%) | 46/637 (7.2%) | 0.85 (0.56; 1.29) |
| Above median | 34/637 (5.3%) | 44/664 (6.6%) | 0.80 (0.51; 1.25) |

| | | | |
|---|---|---|---|
| CABG denotes coronary artery bypass graft, MI myocardial infarction, PCI percutaneous coronary intervention, and TIA transient ischemic attack. †The final amendment to the statistical analysis plan, which listed the subgroups of interest, was approved on August 28, 2019 and unblinding occurred on August 29, 2019. ‡The hazard ratio for the primary endpoint was 0.70 (0.52; 0.93) in men and 0.81 (0.47; 1.41) in women in the per-protocol population. The median value for total white blood cell count was 8.64 X 10³/µL. | | | |

**Table 6. Total (First and Recurrent) Primary Endpoint Events.**

| **Total Primary Endpoint Events** | | **Colchicine (N=2366)** | **Placebo (N=2379)** | |
|---|---|---|---|---|
| Number of primary endpoint events per patient | 0 | 2235 | 2209 | |
| | 1 | 111 | 132 | |
| | 2 | 18 | 26 | |
| | 3 | 1 | 9 | |
| | 4 | 1 | 3 | |
| | | | | |
| Total number of primary endpoint events | | 154 | 223 | |
| Total follow-up months | | 52949 | 53060 | |
| Rate of primary endpoint events per 100 patient-months | | 0.29 | 0.42 | |

| | | | | **Hazard Ratio or Rate Ratio (95% CI)** |
|---|---|---|---|---|
| Negative binomial model† | | | | 0.66 (0.51; 0.86) |
| Andersen-Gill model‡ | | | | 0.69 (0.54; 0.88) |
| WLW model¶ | 1^{st} Event | | | 0.77 (0.61; 0.96) |
| | 2^{nd} Event | | | 0.73 (0.48; 1.11) |
| | 3^{rd} Event | | | 0.64 (0.37; 1.10) |
| | Average | | | 0.77 (0.61; 0.96) |

| | | | | |
|---|---|---|---|---|
| WLW denotes Wei-Lin-Weissfeld method. †The negative binomial regression model was used to calculate marginal rate ratio. ‡The Andersen-Gill model was used with a robust variance estimator (sandwich estimator) to calculate hazard ratio. | | | | |

Regarding Table 6, the Wei-Lin-Weissfeld marginal model was used to calculate hazard ratios for the time to the first, second and third event as well as the weighted average of these hazard ratios.

To account for the occurrence of multiple primary endpoint events within patients, recurrent event analyses were undertaken using three statistical approaches as there is no strong consensus as to which method is preferable. First, a negative binomial regression model was used with the number of events as the outcome and the length of follow-up time in months as an offset term (Hansson GK. Inflammation, atherosclerosis, and coronary artery disease. N Engl J Med 2005;352:1685-95). Marginal rate ratio was provided, along with 95% confidence interval. The Andersen and Gill model (Ridker PM, et al., Antiinflammatory therapy with canakinumab for atherosclerotic disease. N Engl J Med 2017;377:1119-31) with a robust variance estimator (Ridker PM et al., Low-dose methotrexate for the prevention of atherosclerotic events. N Engl J Med 2019;380:752-62; and Ravelli RB et al., Insight into tubulin regulation from a complex with colchicine and a stathmin-like domain. Nature 2004;428:198-202) was utilized to account for the dependency of within-patient events based on a gap-time approach considering the time since a previous event. The Andersen-Gill model is a simple extension of the Cox model based on all events of all patients and estimates a hazard ratio assuming that the instantaneous risk of experiencing an event is the same irrespective of whether previous events occurred. Results from these two models are often similar (Perico N et al., Colchicine interferes with L-selectin and leukocyte function-associated antigen-1 expression on human T lymphocytes and inhibits T cell activation. J Am Soc Nephrol 1996;7:594-601; and Pope RM and Tschopp J, The role of interleukin-1 and the inflammasome in gout: implications for therapy. Arthritis Rheum 2007;56:3183-8). Finally, an approach based on the Wei, Lin and Weissfeld marginal model was conducted whereby times from randomization to first, second and subsequent event were modeled with a Cox proportional hazards model that used a covariance matrix estimate for the regression coefficients that accounted for the possible intra-patient correlation (Cerquaglia C et al., Pharmacological and clinical basis of treatment of Familial Mediterranean Fever (FMF) with colchicine or analogues: an update. Curr Drug Targets Inflamm Allergy 2005;4:117-24; Imazio M et al., Colchicine in addition to conventional therapy for acute pericarditis: results of the COlchicine for acute PEricarditis (COPE) trial. Circulation 2005;112:2012-6; and Nidorf SM et al., Low-dose colchicine for secondary prevention of cardiovascular disease. J Am Coll Cardiol 2013;61:404-10).This approach assumes that all patients are at risk for any event since randomization. Marginal hazard ratios for a k^{th} event (i.e. based on time from randomization to k^{th} event), as well as a weighted average of these hazard ratios, were provided along with 95% confidence intervals. It has been argued that this approach preserves the randomization and permits valid treatment effect estimation (Fine JP and Gray RJ, A proportional hazards model for the subdistribution of a competing risk. J Am Stat Assoc 1999;94:496-509).

### Biomarkers of Inflammation

High-sensitivity C-reactive protein was measured in a subgroup of only 207 patients at the time of randomization and 6 months later, and the median concentration at trial entry was 4.28 mg per liter. The baseline characteristics of these patients were similar to those of the overall population (Table 7), but the small and selected subgroup with these data limits the interpretation of these analyses. The adjusted geometric mean percent changes in the high-sensitivity C-reactive protein level at 6 months after myocardial infarction were -70.0% in the colchicine group and -66.6% in the placebo group, and the placebo-adjusted geometric mean percent change was -10.1% percentage points in the colchicine group (95% Cl, -28.6 to 13.4) (Table 8).

**Table 7. Characteristics of the Trial Patients with hs-CRP data values.**

| **Characteristic** | **Colchicine (N=99)** | **Placebo (N=108)** |
|---|---|---|
| Age - years | 62.1±9.7 | 61.2±10.2 |
| Female sex - no. (%) | 18 (18.2%) | 14 (13.0%) |
| Caucasian - no. (%) | 91 (93.8%) | 89 (89.0%) |
| Body-mass index (kg/m²) | 28.8±4.5 | 29.1±4.2 |
| Smoking - no. (%) | 20 (20.2%) | 20 (18.5%) |
| Hypertension - no. (%) | 44 (44.4%) | 62 (57.4%) |
| Diabetes - no. (%) | 12 (12.1%) | 15 (13.9%) |
| Prior MI- no. (%) | 14 (14.1%) | 16 (14.8%) |
| Prior PCI - no. (%) | 24 (24.2%) | 21 (19.4%) |
| Prior CABG - no. (%) | 4 (4.0%) | 6 (5.6%) |
| Prior heart failure - no. (%) | 4 (4.0%) | 1 (0.9%) |
| Prior stroke/TIA - no. (%) | 1 (1.0%) | 2 (1.9%) |
| Index MI to randomization - days | 17.0±9.2 | 15.8±9.8 |
| PCI for index MI - no. (%) | 93 (93.9%) | 104 (96.3%) |
| Aspirin use - no. (%) | 98 (99.0%) | 106 (98.1%) |
| Other anti-platelet agent - no. (%) | 98 (99.0%) | 108 (100%) |
| Statin use - no. (%) | 99 (100%) | 107 (99.1%) |
| Beta-blocker - no. (%) | 83 (83.8%) | 86 (79.6%) |

| | | |
|---|---|---|
| CABG denotes coronary artery bypass graft surgery, MI myocardial infarction, PCI percutaneous coronary intervention, and TIA transient ischemic attack. | | |

**Table 8. Biomarkers of Inflammation.**

| **Biomarker** | **Colchicine** | **Placebo** |
|---|---|---|
| Hs-C reactive protein (mg/L) | N=99 | N=108 |
| Randomization, geometric mean (IQR)† | 4.27 (2.12, 7.22) | 5.09 (2.45, 11.96) |
| 6 months, geometric mean (IQR) | 1.37 (0.75, 2.13) | 1.60 (0.90, 2.65) |
| Adjusted GM percent change (95% Cl)‡ | -70.0 (-74.6, -64.5) | -66.6 (-71.5, -60.8) |
| Placebo-adjusted GM percent change (95% CI)¶ | -10.1 (-28.6, 13.4) | -- |
| | | |
| Total white blood cell count (10³/µL) | N=992 | N=980 |
| Randomization, geometric mean (IQR)† | 8.54 (7.10, 10.40) | 8.63 (7.20, 10.70) |
| 12 months, geometric mean (IQR) | 6.95 (5.99, 8.30) | 7.03 (5.96, 8.48) |
| Adjusted GM percent change (95% Cl)‡ | -18.81 (-20.12, -17.47) | -19.02 (-20.46, -17.55) |
| Placebo-adjusted GM percent change (95% CI)¶ | 0.26 (-2.15, 2.72) | -- |
| | | |
| Circulating lymphocytes (10³/µL) | | |
| Randomization, geometric mean (IQR)† | 1.79 (1.40, 2.40) | 1.79 (1.42, 2.46) |
| 12 months, geometric mean (IQR) | 1.83 (1.50, 2.44) | 1.82 (1.50, 2.44) |
| Adjusted GM percent change (95% Cl)‡ | 1.80 (-0.46, 4.11) | 0.69 (-1.54, 2.98) |
| Placebo-adjusted GM percent change (95% CI)¶ | 1.10 (-2.06, 4.36) | -- |
| | | |
| Circulating neutrophils (10³/µL) | | |
| Randomization, geometric mean (IQR)† | 5.45 (4.36, 7.15) | 5.47 (4.30, 7.46) |
| 12 months, geometric mean (IQR) | 3.95 (3.27, 5.08) | 3.99 (3.34, 5.20) |
| Adjusted GM percent change (95% Cl)‡ | -27.63 (-29.48, -25.73) | -27.95 (-29.91, -25.93) |
| Placebo-adjusted GM percent change (95% CI)¶ | 0.45 (-3.28, 4.32) | -- |

| | | |
|---|---|---|
| GM denotes geometric mean, HS high-sensitivity, and IQR inter-quartile range. †The geometric mean was obtained by exponentiating the mean of log-transformed data. ‡ The adjusted geometric mean percent change was obtained by exponentiating the adjusted mean from the analysis of covariance model (based on log-transformed data), then subtracting 1 and multiplying by 100. The bounds of the 95% confidence intervals were obtained similarly. | | |

In Table 8, the placebo-adjusted geometric mean percent change was obtained by exponentiating the adjusted mean difference between groups from the analysis of covariance model (based on log-transformed data), then subtracting 1 and multiplying by 100.

In addition, the C-reactive protein biomarker sub-study was implemented following a protocol amendment and was optional for sites and for patients; 34 sites accepted to participate in this substudy. There were 213 and 208 patients who provided blood samples at baseline and 6 months, respectively. Paired baseline and 6-month hs-CRP values were available in 207 patients. Clinically available white blood cell counts were obtained from 2598 patients at baseline and 1998 patients at 12 months, and paired baseline and 12-month values were available in 1972 patients. Statistical analysis was conducted on the patients who provided both baseline and follow-up data and as these were exploratory analyses, no missing data was imputed.

Information about white-cell counts at baseline and at the 12-month follow-up were also available for a relatively small subgroup of 1972 patients. The adjusted geometric mean percent changes from baseline to 1 year in the total white-cell count were -18.8% in the colchicine group and -19.0% in the placebo group, with no significant difference between groups (0.3% percentage points; 95% Cl, -2.2 to 2.7).

### Safety and Adverse Events

The incidence of adverse events that were considered to be related to trial drug was 16.0% in the colchicine group and 15.8% in the placebo group, and the overall incidence of serious adverse events was 16.4% and 17.2%, respectively (Table 9).

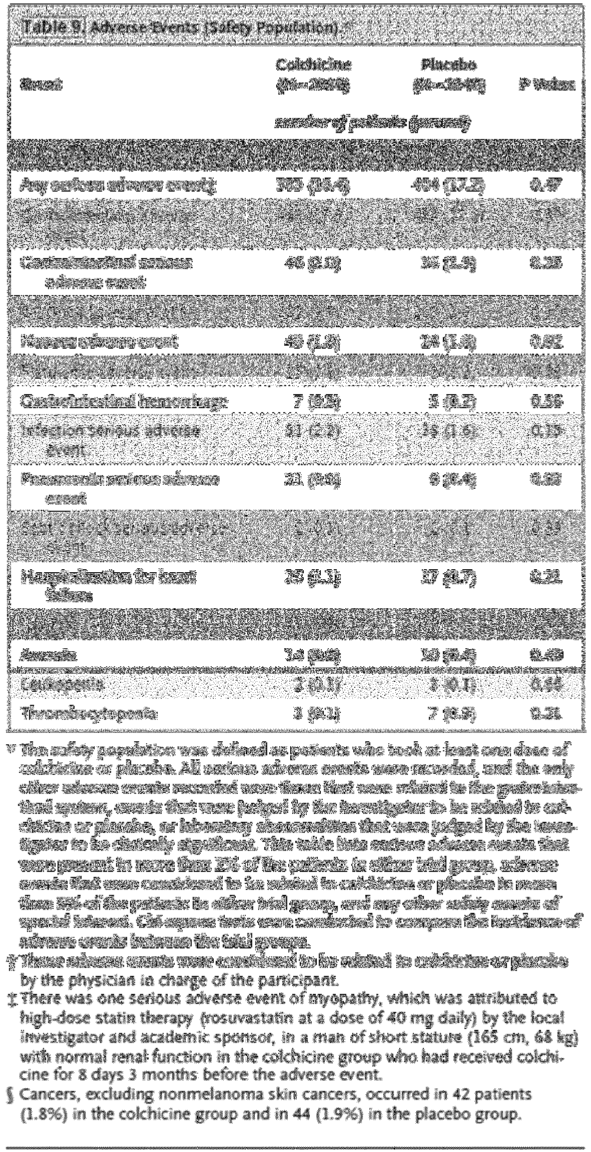

At least one gastrointestinal adverse event during the double-blind period occurred in 17.5% of the patients in the colchicine group, as compared with 17.6% of those in the placebo group. Diarrhea was reported in 9.7% of the patients in the colchicine group and in 8.9% of those in the placebo group (P = 0.35), and nausea was more common in the colchicine group than in the placebo group (1.8% vs. 1.0%, P = 0.02). Pneumonia was reported as a serious adverse event in 0.9% of the patients in the colchicine group, as compared with 0.4% of those in the placebo group (P = 0.03).

In COLCOT, the risk of the primary composite efficacy end point of death from cardiovascular causes, resuscitated cardiac arrest, myocardial infarction, stroke, or urgent hospitalization for angina leading to coronary revascularization, as assessed in a time-to-event analysis, was significantly lower among the patients who were randomly assigned to receive 0.5 mg of colchicine once daily than among those who received placebo. This result was due predominantly to a lower incidence of strokes and urgent hospitalizations for angina leading to coronary revascularization.

These results were observed against a background of appropriate medications, which included aspirin, a different antiplatelet agent, and a statin in 98 to 99% of the patients. In addition, percutaneous coronary intervention was performed in 93% of the patients for their index myocardial infarction. The benefits of colchicine with regard to cardiovascular end points in COLCOT were at least as large as those of canakinumab in CANTOS (Ridker PM et al., Antiinflammatory therapy with canakinumab for atherosclerotic disease. N Engl J Med 2017;377:1119-31). In the small subgroup of patients with available data, a large (>65%) reduction in the C-reactive protein level occurred over the first 6 months after myocardial infarction in both trial groups in COLCOT, but the difference between the changes in the groups was not significant. These findings must be interpreted cautiously given that this was a small subgroup that was not randomly selected from the full trial sample. A similar observation was made with white-cell counts. The different patient populations involved in the two trials - early after myocardial infarction in COLCOT and stable coronary disease in CANTOS - may also have affected the relationship between biomarkers of inflammation and the effects of treatments on ischemic end points.

The known benefits of colchicine in the treatment of pericarditis were not at play in COLCOT. Postinfarction pericarditis typically occurs within the first few days after the injury, whereas the mean time from the index myocardial infarction to randomization was 13.5 days. There were only two patients with a first positively adjudicated event of urgent hospitalization for angina leading to coronary revascularization within 14 days after randomization, and the median time to this clinical end point was 258 days.

The most common adverse events observed were gastrointestinal. Diarrhea was reported in 9.7% of the patients in the colchicine group and in 8.9% of those in the placebo group, and nausea occurred in 1.8% and 1.0%, respectively. Infection as a serious adverse event was more frequent in the colchicine group than in the placebo group (in 2.2% vs. 1.6% of the patients), and pneumonia as a serious adverse event was also more frequent in the colchicine group (0.9% vs. 0.4%). These differences in the incidence of infections could be due to the play of chance or could reflect altered immunologic responses.

In contrast to canakinumab (Ridker PM et al., Antiinflammatory therapy with canakinumab for atherosclerotic disease. N Engl J Med 2017;377:1119-31), colchicine did not increase the incidence of septic shock in our trial. Infections have previously been described in patients who have attempted suicide by taking an overdose of colchicine (Kocak Z et al., Colchicine intoxication and infection risk: a case report. J Clin Pharm Ther 2008;33:451-2). There was no serious adverse event of myopathy linked to colchicine despite the use of statins in 99% of the patients in the trial.

Our trial has certain limitations. The duration of follow-up was relatively short at approximately 23 months. The risks and benefits of longer-term treatment with colchicine were not evaluated. Although the inclusion of 4745 patients was sufficient for the trial to show a significant benefit with regard to the primary composite efficacy end point, a larger trial could have allowed a better assessment of individual end points and subgroups and the risks associated with colchicine. Finally, our results apply only to patients who have recently had a myocardial infarction.

In conclusion, among patients with a recent myocardial infarction, colchicine at a dose of 0.5 mg daily led to a significantly lower percentage of patients with ischemic cardiovascular events than placebo.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features hereinbefore set forth, and follows in the scope of the claims.

Other embodiments are within the claims.

## Claims

1. Colchicine for use in a method of treating a patient after having a myocardial infarction (MI), the method comprising initiating the administration of colchicine at a daily low dose to the patient within about 30 days of the MI.

2. Colchicine for use according to claim 1, wherein the method comprises administering colchicine within 5, 10, 15, 20, or 25 days of the MI.

3. Colchicine for use according to claim 2, wherein the method comprises administering colchicine within 10 days of the MI.

4. Colchicine for use according to claim 2, wherein the method comprises administering colchicine within 15 days of the MI.

5. Colchicine for use according to any one of claims 1-4, wherein percutaneous coronary intervention was performed for treating the patient's MI.

6. Colchicine for use according to any one of claims 1-5, wherein the patient was prescribed a medication.

7. Colchicine for use according to claim 6, wherein the medication is an antiplatelet agent.

8. Colchicine for use according to claim 6, wherein the medication is aspirin.

9. Colchicine for use according to claim 6, wherein the medication is a statin.

10. Colchicine for use according to any one of claims 1-9, wherein the patient is at a lower risk of an ischemic cardiovascular event.

11. Colchicine for use according to any one of claims 1-10, wherein the administration of colchicine is initiated upon assessment in (a) an emergency department (ED), (b) the hospital, or (c) a medical office setting.

12. Colchicine for use according to any one of claims 1-11, wherein the colchicine is in the form of a tablet.

13. Colchicine for use according to any one of claims 1-12, wherein the colchicine is administered at 0.3 to 0.7 mg.

14. Colchicine for use according to any one of claims 1-13, wherein the colchicine is administered at 0.4 to 0.6 mg, preferably 0.5 mg.

15. Colchicine for use according to any one of claims 1-14, wherein the colchicine is administered once, twice or three times a day.
